# EUROPÄISCHE PATENTSCHRIFT

(11) **EP 1 064 262 B1**
(45) Veröffentlichungstag und Bekanntmachung des Hinweises auf die Patenterteilung: **12.02.2003**
(21) Anmeldenummer: 99907548.4
(22) Anmeldetag: 12.02.1999
(51) Int. Cl.: C07D 209/48, A61K 31/40, C07D 401/04, C07D 409/04

(54) **SUBSTITUIERTE ISOINDOLONE UND IHRE VERWENDUNG ALS CYCLISCHE GMP MODULATOREN IN ARZNEIMITTELN**
SUBSTITUTED ISOINDOLONES AND THEIR USE AS CYCLIC GMP MODULATORS IN MEDICAMENTS
ISOINDOLONES SUBSTITUEES ET LEUR UTILISATION COMME MODULATEURS DU GMP CYCLIQUE

(30) Priorität: 21.02.1998 DE 19807423
(43) Veröffentlichungstag der Anmeldung: 03.01.2001
(73) Patentinhaber: Aventis Pharma Deutschland GmbH, 65929 Frankfurt am Main (DE)
(72) Erfinder: SCHINDLER, Ursula, D-65812 Bad Soden (DE); SCHÖNAFINGER, Karl, D-63755 Alzenau (DE); STROBEL, Hartmut, D-65835 Liederbach (DE); GROEHN, Viola, CH-8212 Neuhausen (CH)
(86) Internationale Anmeldenummer: EP9900931
(87) Internationale Veröffentlichungsnummer: WO99042444

(56) Entgegenhaltungen:
- EP-A- 0 667 345
- EP-A- 0 743 304

## Beschreibung

Die vorliegende Erfindung betrifft substituierte Isoindolon-Derivate der Formel I, 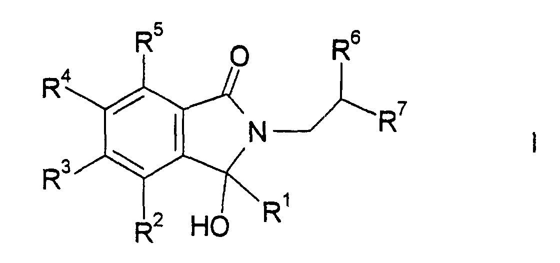 in der R¹, R², R³, R⁴, R⁵, R⁶ und R⁷ die unten angegebenen Bedeutungen haben, die wertvolle Arzneimittelwirkstoffe für die Therapie und Prophylaxe von Krankheiten sind, zum Beispiel von Herz-Kreislauferkrankungen wie Bluthochdruck, Angina pectoris, Herzinsuffizienz, Thrombosen oder Atherosklerose. Die Verbindungen der Formel I haben die Fähigkeit zur Modulation der körpereigenen Produktion von cyclischem Guanosinmonophosphat (cGMP) und eignen sich generell zur Therapie und Prophylaxe von Krankheitszuständen, die mit einem gestörten cGMP-Haushalt verbunden sind. Die Erfindung betrifft weiterhin Verfahren zur Herstellung von Verbindungen der Formel I, ihre Verwendung zur Therapie und Prophylaxe der bezeichneten Krankheitszustände und zur Herstellung von Arzneimitteln dafür sowie pharmazeutische Präparate, die Verbindungen der Formel I enthalten.

cGMP ist ein wichtiger intrazellulärer Botenstoff, der über die Modulation von cGMP-abhängigen Proteinkinasen, Phosphodiesterasen und lonenkanälen eine Reihe von pharmakologischen Effekten auslöst. Beispiele sind die Glattmuskelrelaxation, die Inhibition der Thrombozytenaktivierung und die Hemmung von Glattmuskelzellproliferation und Leukozytenadhäsion. cGMP wird durch partikuläre und lösliche Guanylatcyclasen (GC) als Antwort auf eine Reihe extrazellulärer und intrazellulärer Stimuli produziert. Im Falle der partikulären Guanylatcyclasen erfolgt die Stimulation im wesentlichen durch peptidische Signalstoffe, wie dem atrialen natriuretischen Peptid oder dem cerebralen natriuretischen Peptid. Die löslichen Guanylatcyclasen (sGC), bei denen es sich um cytosolische, heterodimere Hämproteine handelt, werden dagegen im wesentlichen durch eine Familie niedermolekularer, enzymatisch gebildeter Faktoren reguliert. Wichtigstes Stimulans ist das Stickstoffmonoxid (NO) oder eine nahe verwandte Spezies. Die Bedeutung anderer Faktoren wie Kohlenmonoxid oder dem Hydroxylradikal ist noch weitgehend ungeklärt. Als Aktivierungsmechanismus der Aktivierung durch NO wird die Anbindung von NO an das Häm unter Ausbildung eines pentakoordinierten Häm-Nitrosyl-Komplexes diskutiert. Die damit verbundene Freisetzung des im Basal-Zustand an das Eisen gebundenen Histidins überführt das Enzym in die aktivierte Konformation.

Aktive lösliche Guanylatcyclasen setzen sich aus je einer α- und einer β-Untereinheit zusammen. Von den Untereinheiten wurden mehrere Subtypen beschrieben, die sich untereinander bezüglich Sequenz, gewebespezifischer Verteilung und Expression in verschiedenen Entwicklungsstadien unterscheiden. Die Subtypen α₁ und β₁ werden hauptsächlich in Gehirn und Lunge exprimiert, während β₂ vor allem in Leber und Niere gefunden wird. In humanem fötalen Gehirn konnte der Subtyp α₂ nachgewiesen werden. Die als α₃ und β₃ bezeichneten Untereinheiten wurden aus menschlichem Gehirn isoliert und sind homolog zu α₁ und β₁. Neuere Arbeiten weisen auf eine α₂ᵢ-Untereinheit hin, die ein Insert in der katalytischen Domäne enthält. Alle Untereinheiten zeigen große Homologien im Bereich der katalytischen Domäne. Die Enzyme enthalten vermutlich ein Häm pro Heterodimer, das über β₁-Cys-78 und/oder β₁-His-105 gebunden ist und Teil des regulatorischen Zentrums ist.

Unter pathologischen Bedingungen kann die Bildung Guanylatcyclaseaktivierender Faktoren vermindert sein oder es kann durch das vermehrte Auftreten freier Radikale ein verstärkter Abbau derselben erfolgen. Die daraus resultierende verminderte Aktivierung der sGC führt über die Abschwächung der jeweiligen cGMP-vermittelten Zellantwort beispielsweise zum Anstieg des Blutdrucks, zur Plättchenaktivierung oder zu vermehrter Zellproliferation und Zelladhäsion. Als Folge kommt es zur Ausbildung von endothelialer Dysfunktion, Atherosklerose, Bluthochdruck, stabiler und instabiler Angina pectoris, Thrombosen, Myocardinfarkt, Schlaganfällen oder erektiler Dysfunktion. Die pharmakologische Stimulation der sGC bietet eine Möglichkeit zur Normalisierung der cGMP-Produktion und erlaubt damit die Behandlung und Prävention derartiger Krankheiten.

Zur pharmakologischen Stimulation der sGC wurden bisher fast ausschließlich Verbindungen verwendet, deren Wirkung auf einer intermediären NO-Freisetzung beruht, beispielsweise organische Nitrate. Der Nachteil dieser Behandlungsweise liegt in der Toleranzentwicklung und Wirkungsabschwächung und der deshalb erforderlich werdenden höheren Dosierung.

Verschiedene nicht über eine NO-Freisetzung wirkende sGC-Stimulatoren wurden von Vesely in einer größeren Zahl von Arbeiten beschrieben. Die Verbindungen, bei denen es sich zumeist um Hormone, Pflanzenhormone, Vitamine oder zum Beispiel Naturstoffe wie Echsengifte handelt, zeigen jedoch durchweg nur schwache Effekte auf die cGMP-Bildung in Zellysaten (D. L. Vesely, Eur. J. Clin. Invest. 15 (1985) 258; D. L. Vesely, Biochem. Biophys. Res. Comm. 88 (1979) 1244). Eine Stimulation Häm-freier Guanylatcyclase durch Protoporphyrin IX wurde durch Ignarro et al. (Adv. Pharmacol. 26 (1994) 35) nachgewiesen. Pettibone et al. (Eur. J. Pharmacol. 116 (1985) 307) beschrieben für Diphenyliodoniumhexafluorophoshat eine blutdrucksenkende Wirkung und führten diese auf eine Stimulation der sGC zurück. Isoliquiritiginin, das an isolierten Rattenaorten eine relaxierende Wirkung zeigt, aktiviert laut Yu et al. (Brit. J. Pharmacol. 114 (1995) 1587) ebenfalls die sGC. Ko et al. (Blood 84 (1994) 4226), Yu et al. (Biochem. J. 306 (1995) 787) und Wu et al. (Brit. J. Pharmacol. 116 (1995) 1973) wiesen eine sGC-stimulierende Aktivität von 1-Benzyl-3-(5-hydroxymethyl-2-furyl)-indazol nach und demonstrierten eine antiproliferative und thrombozytenhemmende Wirkung. Verschiedene Indazole werden in EP-A-667 345 als Inhibitoren der Thrombozytenaggregation beschrieben.

Verschiedene am Stickstoff substituierte 3-Hydroxyisoindol-1-one sind bekannt. Winn und Zaugg beschreiben in J. Org. Chem. 33 (1968) 3779 die Verbindungen der Formel I, in der R¹ für unsubstituiertes Phenyl steht, R², R³, R⁴, R⁵ und R⁷ für Wasserstoff stehen und R⁶ für unsubstituiertes Phenyl oder für 3,4-Dimethoxyphenyl steht. Über eine pharmakologische Aktivität der Verbindungen wird aber nichts berichtet. Überraschend wurde nun gefunden, daß die erfindungsgemäßen Verbindungen der Formel I eine starke Guanylatcyclase-Aktivierung bewirken, aufgrund derer sie zur Therapie und Prophylaxe von Krankheiten geeignet sind, die mit einem niedrigen cGMP-Spiegel verbunden sind.

Die vorliegende Erfindung betrifft somit Verbindungen der Formel I, 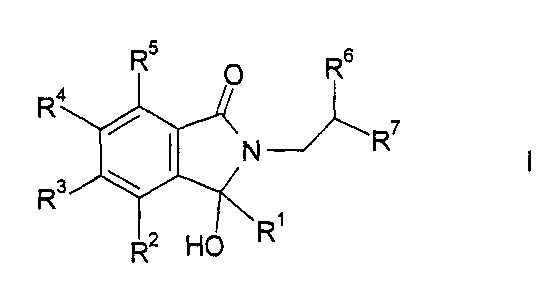 in der
R¹ für (C₃-C₇)-Cycloalkyl, Phenyl oder den Rest eines 5-gliedrigen oder 6-gliedrigen aromatischen Heterocyclus, der ein oder zwei gleiche oder verschiedene Heteroatome aus der Reihe N, O, S enthält, steht, wobei der Phenylrest und der heterocyclische Rest unsubstituiert sind oder durch einen oder mehrere gleiche oder verschiedene Reste aus der Reihe (C₁-C₄)-Alkyl, Halogen, Hydroxy, (C₁-C₄)-Alkoxy, Benzyloxy, Phenoxy, Benzyl, Phenyl, Trifluormethyl, Cyan, Hydroxycarbonyl, ((C₁-C₄)-Alkoxy)carbonyl, Aminocarbonyl, Nitro, Amino, (C₁-C₄)-Alkylamino, Di-((C₁-C₄)-alkyl)amino und ((C₁-C₄)-Alkyl)carbonylamino substituiert sind;
R², R³, R⁴ und R⁵ unabhängig voneinander für Wasserstoff, Halogen, (C₁-C₄)-Alkyl oder (C₁-C₄)-Alkoxy stehen;
R⁶ für Phenyl steht, das unsubstituiert ist oder durch einen oder mehrere gleiche oder verschiedene Reste aus der Reihe (C₁-C₄)-Alkyl, Halogen, Hydroxy, (C₁-C₄)-Alkoxy, Benzyloxy, Phenoxy, Benzyl, Phenyl, Trifluormethyl, Cyan, Hydroxycarbonyl, ((C₁-C₄)-Alkoxy)carbonyl, Aminocarbonyl, Nitro, Amino, (C₁-C₄)-Alkylamino, Di-((C₁-C₄)-alkyl)amino und ((C₁-C₄)-Alkyl)carbonylamino substituiert ist;
R⁷ unabhängig von R⁶ eine der Bedeutungen von R⁶ hat oder für Wasserstoff steht;
in allen ihren stereoisomeren Formen und Mischungen davon in allen Verhältnissen, und ihre physiologisch verträglichen Salze;
wobei, wenn R¹ für unsubstituiertes Phenyl steht und R², R³, R⁴, R⁵ und R⁷ für Wasserstoff stehen, dann nicht gleichzeitig R⁶ für unsubstituiertes Phenyl oder für 3,4-Dimethoxyphenyl stehen kann.

Alkylreste können geradkettig oder verzweigt sein. Dies gilt auch, wenn sie in anderen Gruppen enthalten sind, zum Beispiel in Alkoxygruppen. Beispiele für Alkylgruppen sind Methyl, Ethyl, n-Propyl, Isopropyl, n-Butyl, Isobutyl, sec-Butyl und tert-Butyl. Unter dem Begriff Alkyl sind hier auch ungesättigte Alkylreste zu verstehen, insbesondere Alkylreste, die eine Doppelbindung oder eine Dreifachbindung enthalten. Beispiele für solche Reste sind der Vinylrest, der 2-Propenylrest (Allylrest), der 2-Butenylrest, der 2-Methyl-2-propenylrest, der Ethinylrest, der 2-Propinylrest (Propargylrest) oder der 3-Butinylrest.

Beispiele für Cycloalkylreste sind Cyclopropyl, Cyclobutyl, Cyclopentyl, Cyclohexyl und Cycloheptyl, die alle auch zum Beispiel durch einen oder mehrere gleiche oder verschiedene (C₁-C₄)-Alkylreste, insbesondere durch Methyl, substituiert sein können. Beispiele für substituierte Cycloalkylreste sind 4-Methylcyclohexyl, 4-tert-Butylcyclohexyl oder 2,3-Dimethylcyclopentyl.

Phenylreste können unsubstituiert sein oder einen oder mehrere, zum Beispiel zwei, drei oder vier, gleiche oder verschiedene Substituenten tragen. Die Substituenten können sich in beliebigen Positionen befinden. Bevorzugt sind substituierte Phenylreste einfach oder zweifach substituiert. Monosubstituierte Phenylreste können in der 2-Position, der 3-Position oder der 4-Position substituiert sein, in disubstituierten Phenylresten können sich die Substituenten in 2,3-Position, 2,4-Position, 2,5-Position, 2,6-Position, 3,4-Position oder 3,5-Position befinden. In trisubstituierten Phenylresten können sich die Substituenten in 2,3,4-Position, 2,3,5-Position, 2,3,6-Position, 2,4,5-Position, 2,4,6-Position oder 3,4,5-Position befinden.

Beispiele für 5-gliedrige und 6-gliedrige aromatische Heterocyclen sind Furan, Thiophen, Pyrrol, Pyrazol, Imidazol, 1,3-Oxazol, 1,3-Thiazol, Pyridin, Pyridazin, Pyrimidin und Pyrazin. Die von diesen Heterocyclen abgeleiteten Reste können über jedes Kohlenstoffatom gebunden sein. Beispielsweise kann ein Thienylrest als 2-Thienylrest oder 3-Thienylrest vorliegen, ein Furylrest als 2-Furylrest oder 3-Furylrest, ein Pyridylrest als 2-Pyridylrest, 3-Pyridylrest oder 4-Pyridylrest. Die Heterocyclen können unsubstituiert sein oder einen oder mehrere, zum Beispiel zwei, drei oder vier, gleiche oder verschiedene Substituenten tragen. Bevorzugt sind heterocyclische Reste unsubstituiert oder durch einen oder zwei gleiche oder verschiedene Reste substituiert, insbesondere unsubstituiert. Die Substituenten in Heterocyclen können sich in beliebigen Positionen befinden, beispielsweise in einem über die 2-Position gebundenen Thienylrest oder Furylrest in der 3-Position, der 4-Position und/oder der 5-Position, in einem über die 3-Position gebundenen Thienylrest oder Furylrest in der 2-Position, der 4-Position und/oder der 5-Position, in einem 2-Pyridylrest in der 3-Position, der 4-Position, der 5-Position und/oder der 6-Position, in einem 3-Pyridylrest in der 2-Position, der 4-Position, der 5-Position und/oder der 6-Position, in einem 4-Pyridylrest in der 2-Position, der 3-Position, der 5-Position und/oder der 6-Position. Bevorzugte Substituenten in heterocyclischen Resten sind (C₁-C₄)-Alkylreste, insbesondere Methyl, und/oder Halogenatome, insbesondere Chlor und/oder Fluor. Stickstoffheterocyclen können auch als N-Oxide oder als Quartärsalze vorliegen, zum Beispiel Pyridylreste als Pyridin-N-oxide.

Sind in Phenylresten und/oder heterocyclischen Resten Nitrogruppen als Substituenten vorhanden, so können in den Verbindungen der Formel I insgesamt nur bis zu zwei Nitrogruppen im Molekül vorhanden sein. Sind in Phenylresten und/oder in heterocyclischen Resten Phenylreste, Phenoxyreste, Benzylreste oder Benzyloxyreste als Substituenten vorhanden, so kann in diesen der Benzolring auch wiederum unsubstituiert sein oder durch einen oder mehrere, zum Beispiel zwei, drei oder vier, gleiche oder verschiedene Reste aus der Reihe (C₁-C₄)-Alkyl, Halogen, Hydroxy, (C₁-C₄)-Alkoxy, Trifluormethyl, Cyan, Hydroxycarbonyl, ((C₁-C₄)-Alkoxy)carbonyl, Aminocarbonyl, Nitro, Amino, (C₁-C₄)-Alkylamino, Di-((C₁-C₄)-alkyl)amino und ((C₁-C₄)-Alkyl)carbonylamino substituiert sein.

Halogen bedeutet Fluor, Chlor, Brom oder lod, vorzugsweise Fluor oder Chlor.

Die vorliegende Erfindung umfaßt alle stereoisomeren Formen der Verbindungen der Formel I. In den Verbindungen der Formel I enthaltene Asymmetriezentren können alle unabhängig voneinander die S-Konfiguration oder die R-Konfiguration aufweisen. Zur Erfindung gehören alle möglichen Enantiomeren und Diastereomeren, ebenso Mischungen von zwei oder mehr stereoisomeren Formen, zum Beispiel Mischungen von Enantiomeren und/oder Diastereomeren, in allen Verhältnissen. Enantiomere sind also in enantiomerenreiner Form, sowohl als linksdrehende als auch als rechtsdrehende Antipoden, in Form von Racematen und in Form von Mischungen der beiden Enantiomeren in allen Verhältnissen Gegenstand der Erfindung. Bei Vorliegen einer cis/trans-Isomerie sind sowohl die cis-Form als auch die trans-Form und Gemische dieser Formen in allen Verhältnissen Gegenstand der Erfindung. Die Herstellung von einzelnen Stereoisomeren kann gewünschtenfalls durch Auftrennung eines Gemisches nach üblichen Methoden, zum Beispiel durch Chromatographie oder Kristallisation, durch Verwendung von stereochemisch einheitlichen Ausgangssubstanzen bei der Synthese oder durch stereoselektive Synthese erfolgen. Gegebenenfalls kann vor einer Trennung von Stereoisomeren eine Derivatisierung erfolgen. Die Trennung eines Stereoisomerengemisches kann auf der Stufe der Verbindungen der Formel I erfolgen oder auf der Stufe eines Zwischenprodukts im Verlaufe der Synthese. Bei Vorliegen von beweglichen Wasserstoffatomen umfaßt die vorliegende Erfindung auch alle tautomeren Formen der Verbindungen der Formel I. Weiterhin umfaßt sie auch alle Ring-Ketten-Tautomeren der Verbindungen der Formel I, zum Beispiel die Verbindungen der Formel la.

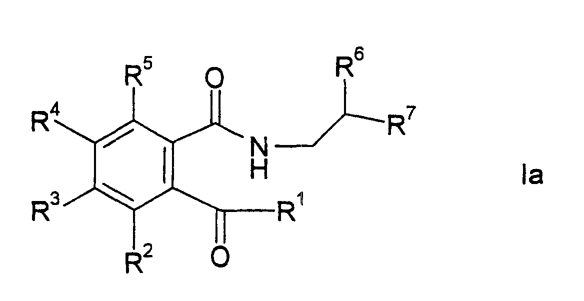

Enthalten die Verbindungen der Formel I eine oder mehrere saure oder basische Gruppen, so sind auch die entsprechenden physiologisch oder toxikologisch verträglichen Salze Gegenstand der Erfindung, insbesondere die pharmazeutisch verwendbaren Salze. So können die Verbindungen der Formel I, die saure Gruppen enthalten, an diesen Gruppen beispielsweise als Alkalimetallsalze, Erdalkalimetallsalze oder als Ammoniumsalze vorliegen und erfindungsgemäß verwendet werden. Beispiele für solche Salze sind Natriumsalze, Kaliumsalze, Calciumsalze, Magnesiumsalze, Salze mit physiologisch verträglichen quartären Ammoniumionen oder Säureadditionssalze mit Ammoniak oder physiologisch verträglichen organischen Aminen wie beispielsweise Ethylamin, Ethanolamin, Triethanolamin oder Aminosäuren. Verbindungen der Formel I, die eine oder mehrere basische, das heißt protonierbare, Gruppen enthalten, können in Form ihrer Säureadditionssalze mit anorganischen oder organischen Säuren vorliegen und erfindungsgemäß verwendet werden, zum Beispiel als Salze mit Chlorwasserstoff, Bromwasserstoff, Phosphorsäure, Schwefelsäure, Salpetersäure, Methansulfonsäure, p-Toluolsulfonsäure, Naphthalindisulfonsäuren, Oxalsäure, Essigsäure, Weinsäure, Milchsäure, Salicylsäure, Benzoesäure, Ameisensäure, Propionsäure, Pivalinsäure, Diethylessigsäure, Malonsäure, Bernsteinsäure, Pimelinsäure, Fumarsäure, Maleinsäure, Äpfelsäure, Sulfaminsäure, Phenylpropionsäure, Gluconsäure, Ascorbinsäure, Isonicotinsäure, Zitronensäure, Adipinsäure usw. Enthalten die Verbindungen der Formel I gleichzeitig saure und basische Gruppen im Molekül, so gehören neben den geschilderten Salzformen auch innere Salze oder Betaine zu der Erfindung. Salze können aus den Verbindungen der Formel I nach üblichen, dem Fachmann bekannten Verfahren erhalten werden, beispielsweise durch Vereinigung mit einer organischen oder anorganischen Säure oder Base in einem Lösungsmittel oder Dispergiermittel, oder auch durch Anionenaustausch oder Kationenaustausch aus anderen Salzen. Die vorliegende Erfindung umfaßt auch alle Salze der Verbindungen der Formel I, die sich wegen geringer physiologischer Verträglichkeit nicht direkt für eine Verwendung in Arzneimitteln eignen, aber zum Beispiel als Zwischenprodukte für chemische Reaktionen oder für die Herstellung physiologisch verträglicher Salze in Betracht kommen.

Die vorliegende Erfindung umfaßt weiterhin alle Solvate von Verbindungen der Formel I, zum Beispiel Hydrate oder Addukte mit Alkoholen, sowie Derivate der Verbindungen der Formel I wie zum Beispiel Ester, und Pro-Drugs und aktive Metabolite.

R¹ steht bevorzugt für unsubstituiertes Phenyl oder Phenyl, das durch einen oder zwei gleiche oder verschiedene Reste aus der Reihe (C₁-C₄)-Alkyl und Halogen substituiert ist. Besonders bevorzugt steht R¹ für unsubstituiertes Phenyl oder Phenyl, das ein oder zwei gleiche oder verschiedene Halogenatome substituiert ist. Speziell bevorzugt steht R¹ für unsubstituiertes Phenyl oder für Fluorphenyl, insbesondere 4-Fluorphenyl.

R², R³, R⁴ und R⁵ stehen bevorzugt unabhängig voneinander für Wasserstoff, Halogen oder (C₁-C₄)-Alkyl, besonders bevorzugt unabhängig voneinander für Wasserstoff, Fluor, Chlor oder Methyl. In einer speziell bevorzugten Ausführungsform stehen alle Reste R², R³, R⁴ und R⁵ für Wasserstoff.

R⁶ steht bevorzugt für Phenyl, das unsubstituiert ist oder durch einen oder zwei gleiche oder verschiedene Reste aus der Reihe (C₁-C₄)-Alkyl, Halogen und (C₁-C₄)-Alkoxy substituiert ist, insbesondere in den Positionen 2 und/oder 3. Besonders bevorzugt steht R⁶ für Phenyl, das durch einen oder zwei gleiche oder verschiedene Reste aus der Reihe Halogen und (C₁-C₄)-Alkoxy substituiert ist. Speziell bevorzugt steht R⁶ für Methoxyphenyl, insbesondere 3-Methoxyphenyl, oder für Chlorphenyl, insbesondere 2-Chlorphenyl.

R⁷ steht bevorzugt für Wasserstoff.

Bevorzugte Verbindungen der Formel I sind solche, in denen einer oder mehrere der darin enthaltenen Reste bevorzugte Bedeutungen haben, wobei alle Kombinationen von bevorzugten Substituentendefinitionen Gegenstand der vorliegenden Erfindung sind. Auch von allen bevorzugten Verbindungen der Formel I umfaßt die vorliegende Erfindung alle ihre stereoisomeren Formen und Mischungen davon in allen Verhältnissen, und ihre physiologisch verträglichen Salze.

Eine Gruppe von bevorzugten Verbindungen der Formel I bilden solche Verbindungen, in denen R¹ für unsubstituiertes Phenyl oder Fluorphenyl, insbesondere 4-Fluorphenyl, steht; R², R³, R⁴ und R⁵ für Wasserstoff stehen; R⁶ für Methoxyphenyl, insbesondere 3-Methoxyphenyl, oder für Chlorphenyl, insbesondere 2-Chlorphenyl, steht; R⁷ für Wasserstoff steht; in allen ihren stereoisomeren Formen und Mischungen davon in allen Verhältnissen, und ihre physiologisch verträglichen Salze.

Gegenstand der vorliegenden Erfindung sind auch Verfahren zur Herstellung der Verbindungen der Formel I, die im folgenden erläutert sind und nach denen die erfindungsgemäßen Verbindungen erhältlich sind. Die Verbindungen können beispielsweise hergestellt werden, indem man analog Delcey et al., Heterocycles 41 (1995) 1721 Benzoesäuren der Formel II, die in der 2-Position durch eine Ketogruppe substituiert sind, oder aktivierte Derivate der Benzoesäuren der Formel II mit primären Aminen der Formel III umsetzt. In den Formeln II und III haben die Reste R¹, R², R³, R⁴, R⁵, R⁶ und R⁷ die oben angegebenen Bedeutungen.

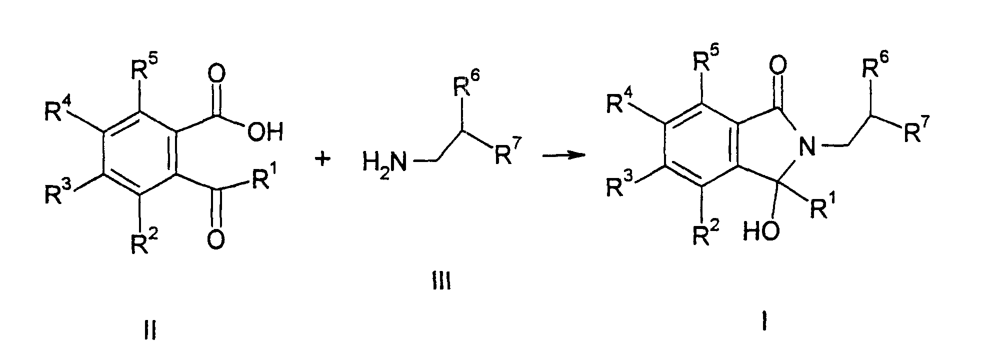

Die als Ausgangssubstanzen eingesetzen 2-Ketobenzoesäuren der Formel II und die Amine der Formel III sind käuflich erhältlich oder können nach oder analog zu in der Literatur beschriebenen Standardverfahren hergestellt werden. Soll die Carbonsäuregruppe in den Verbindungen der Formel II in ein aktiviertes Derivat mit höherer Reaktivität überführt werden, so kann dies nach Standardverfahren erfolgen. Zur Aktivierung kann die Säuregruppe zum Beispiel in einen Ester überführt werden, zum Beispiel in einen Alkylester wie einen Methylester oder Ethylester oder in einen Arylester wie einen Phenylester, Chlorphenylester oder Nitrophenylester, oder sie kann in ein Säurehalogenid wie ein Säurechlorid oder Säurebromid überführt werden, oder sie kann in ein Azolid überführt werden, zum Beispiel mit Carbonyldiimidazol in ein Imidazolid, oder sie kann in ein gemischtes Anhydrid überführt werden, zum Beispiel durch Umsetzung mit einem Chlorameisensäurealkylester. Die Aktivierung der Carbonsäuregruppe für die Umsetzung mit dem Amin der Formel III kann beispielsweise auch mit Aktivierungsreagenzien wie Carbodiimiden oder anderen in der Peptidchemie üblichen Reagenzien erfolgen. Die Aktivierung kann in einem separaten Schritt oder in situ durchgeführt werden. Ein besonders einfaches und günstiges Verfahren ist die Aktivierung von Carbonsäuren der Formel II, insbesondere solchen, in denen R¹ für einen aromatischen Rest, zum Beispiel einen Phenylrest, steht, durch Überführung in die Carbonsäurechloride. Diese Chlorierung der Benzoesäuren zu den Benzoylchloriden kann nach Standardverfahren für die Herstellung von Säurechloriden erfolgen, zum Beispiel unter Verwendung von Thionylchlorid, Phosphorchloriden oder Oxalsäuredichlorid in einem inerten Lösungsmittel oder Dispergiermittel oder auch ohne ein Lösungsmittel.

Die Umsetzung von Verbindungen der Formel II oder deren aktivierten Derivaten mit Verbindungen der Formel III kann nach Standardverfahren für die Umsetzung von Carbonsäuren oder Carbonsäurederivaten mit Aminen unter Reaktionsbedingungen erfolgen, die dem Fachmann wohlbekannt sind. Bevorzugt wird die Umsetzung in einem inerten Lösungsmittel oder Dispergiermittel durchgeführt. Wird beispielsweise nach einer bevorzugten Variante dieses Herstellverfahrens das Säurechlorid einer Verbindung der Formel II eingesetzt, so wird bevorzugt in einem aprotischen Lösungsmittel oder Dispergiermittel gearbeitet. Als Lösungsmittel oder Dispergiermittel kommen in diesem Fall beispielsweise Ether wie Diethylether, Dipropylether, Dibutylether, tert-Butylmethylether, Tetrahydrofuran, Dioxan, Ethylenglykolether und Di- und Triethylenglykolether wie Ethylenglykoldimethylether oder Diethylenglykoldimethylether, Ester wie Essigsäureethylester oder Essigsäurebutylester, Amide wie Dimethylformamid, Dimethylacetamid, N-Methylpyrrolidon oder Hexamethylphosphorsäuretriamid, Nitrile wie Acetonitril, Sulfoxide und Sulfone wie Dimethylsulfoxid oder Sulfolan, Kohlenwasserstoffe und chlorierte Kohlenwasserstoffe wie Benzinfraktionen, Benzol, Toluol, Xylol, Chlorbenzol, Dichlorbenzol, Methylenchlorid oder Chloroform in Betracht. Je nach dem Einzelfall und dem eingesetzten Derivat der Verbindung der Formel II können aber auch andere Lösungsmittel in Betracht kommen, zum Beispiel Wasser oder Alkohole wie Methanol, Ethanol, n-Propanol, Isopropanol oder Butanole. Generell können auch Mischungen von zwei oder mehr Lösungsmitteln eingesetzt werden.

Insbesondere bei der bevorzugten Synthesevariante, bei der das Säurechlorid einer Verbindung der Formel II eingesetzt wird, wird günstig in Gegenwart einer Base, zum Beispiel eines tertiären Amins wie Triethylamin, Ethyldiisopropylamin, N-Methylmorpholin oder Pyridin gearbeitet, die den entstehenden Chlorwasserstoff bindet.

Die Umsetzungen von Verbindungen der Formel II oder deren Derivaten mit den Verbindungen der Formel III zu Verbindungen der Formel I werden im allgemeinen zwischen 0 °C und 140 °C, bevorzugt zwischen Raumtemperatur und 140 °C, durchgeführt, bei der bevorzugten Variante unter Verwendung des Säurechlorids einer Verbindung der Formel II besonders bevorzugt im Temperaturbereich von 70 °C bis 140 °C. Die Reaktionsdauer richtet sich nach dem Einzelfall, im allgemeinen ist die Umsetzung nach 1 bis 10 Stunden beendet. Der Ablauf der Reaktion kann zum Beispiel durch chromatographische Untersuchung des Reaktionsgemisches verfolgt werden. Die entstandenen Verbindungen der Formel I können durch übliche Aufarbeitungsverfahren, zum Beispiel durch Extraktion, aus dem Reaktionsgemisch isoliert werden und gewünschtenfalls durch übliche Reinigungsmethoden, zum Beispiel durch Kristallisation, Sublimation oder durch Chromatographie, gereinigt werden.

In einem weiteren Verfahren zur Herstellung der erfindungsgemäßen Verbindungen werden Phthalimide der Formel IV mit metallorganischen Verbindungen der Formel V umgesetzt, zum Beispiel mit lithiumorganischen Verbindungen oder mit Grignardverbindungen. In den Formeln IV und V haben die Reste R¹, R², R³, R⁴, R⁵, R⁶ und R⁷ die oben angegebenen Bedeutungen, M steht beispielsweise für Li oder für MgCl, MgBr oder Mgl.

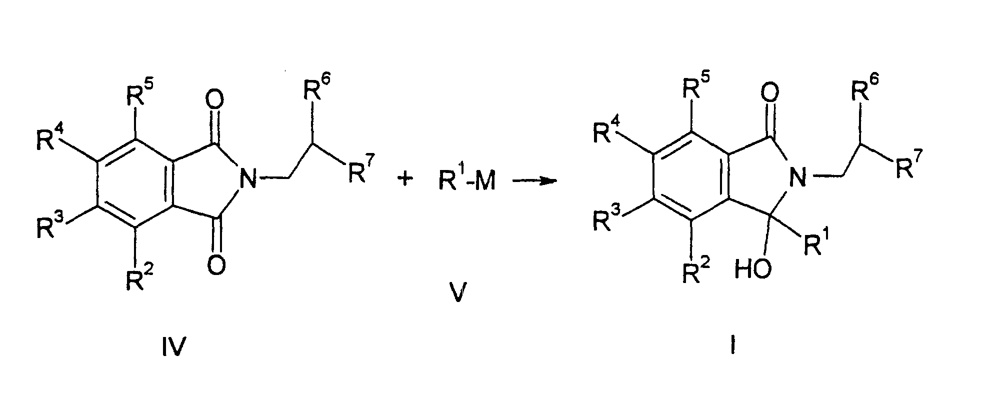

Die Ausgangssubstanzen der Formel IV können nach oder analog zu in der Literatur beschriebenen Standardverfahren hergestellt werden, zum Beispiel nach den Angaben bei Bahajaj und Vernon, J. Chem. Soc. Perkin Trans. 1 (1996) 1041, Boykin et al., J. Heterocycl. Chem. 28 (1991) 609 oder Wanag, Chem. Ber. 75 (1942) 719. Die metallorganischen Verbindungen der Formel V sind käuflich erhältlich oder können nach dem Fachmann wohlbekannten Methoden hergestellt werden, zum Beispiel durch Umsetzung einer organischen Halogenverbindung mit einem Metall wie Magnesium oder Lithium oder durch Ummetallierung.

Die Umsetzung der Phthalimide der Formel IV mit den metallorganischen Verbindungen der Formel V wird im allgemeinen in einem inerten Lösungsmittel oder Dispergiermittel durchgeführt. Bevorzugte Lösungsmittel oder Dispergiermittel sind Ether wie Diethylether, Dipropylether, Dibutylether, tert-Butylmethylether, Tetrahydrofuran, Dioxan, Ethylenglykolether und Di- und Triethylenglykolether wie Ethylenglykoldimethylether oder Diethylenglykoldimethylether. Es können aber auch andere inerte Lösungsmittel verwendet werden, zum Beispiel Kohlenwasserstoffe wie Pentan, Hexan, Heptan, Benzol oder Toluol. Ebenso können auch Mischungen von zwei oder mehr Lösungsmitteln eingesetzt werden.

Die Umsetzung der Verbindungen der Formeln IV und V erfolgt im allgemeinen bei Temperaturen von -75 °C bis +80 °C. Die günstigste Reaktionstemperatur hängt vom Einzelfall ab, zum Beispiel von der Reaktivität der metallorganischen Verbindung. Vielfach ist es günstig, die Reaktionspartner zunächst bei einer tieferen Temperatur zusammenzugeben, zum Beispiel bei ungefähr -70 °C oder bei ungefähr 0 °C, und zur Vervollständigung der Umsetzung das Reaktionsgemisch dann auf eine höhere Temperatur, zum Beispiel Raumtemperatur oder ungefähr 40 °C, zu erwärmen. Die entstandenen Verbindungen der Formel I können wiederum durch übliche Aufarbeitungsverfahren, zum Beispiel wäßrige Aufarbeitung mit Phasentrennung und/oder Extraktion, aus dem Reaktionsgemisch isoliert werden und gewünschtenfalls durch übliche Reinigungsmethoden, zum Beispiel durch Kristallisation, Sublimation oder durch Chromatogrphie, gereinigt werden.

Je nach den Gegebenheiten des Einzelfalles kann es bei der Herstellung der Verbindungen der Formel I nach den angegebenen Verfahren vorteilhaft oder notwendig sein, unter Anwendung der Schutzgruppentechnik zu arbeiten. Welche funktionellen Gruppen vorübergehend zu schützen sind, welche Schutzgruppen dafür geeignet sind und wie die Schutzgruppen wieder entfernt werden können, ist dem Fachmann geläufig und in den Standardwerken der organischen Chemie beschrieben, zum Beispiel in Houben-Weyl, Methoden der Organischen Chemie, Thieme-Verlag, Stuttgart, oder Organic Reactions, John Wiley & Sons, New York. Beispielsweise können Säuregruppen durch Überführung in die Benzylester geschützt werden und die Benzylgruppen später durch katalytische Hydrierung wieder abgespalten werden, oder es können Aminogruppen durch Acylierung geschützt werden. Funktionelle Gruppen können auch zunächst in Form von Vorstufen vorliegen, die dann nach dem Aufbau des Molekülgerüsts der Verbindungen der Formel I nach Standardverfahren in die gewünschten Gruppen umgewandelt werden. Beispielsweise kann eine Nitrogruppe als Vorstufe für eine Aminogruppe dienen.

Die erfindungsgemäßen Verbindungen der Formel I bewirken über die Aktivierung der löslichen Guanylatcyclase (sGC) eine Erhöhung der cGMP-Konzentration und sind deshalb wertvolle Agenzien zur Therapie und Prophylaxe von Krankheiten, die mit einem niedrigen oder erniedrigten cGMP-Spiegel verbunden sind oder durch einen solchen verursacht werden oder zu deren Therapie oder Prophylaxe eine Erhöhung des vorhandenen cGMP-Spiegels angestrebt wird. Die Aktivierung der sGC durch die Verbindungen der Formel I kann zum Beispiel in dem unten beschriebenen Aktivitätsassay untersucht werden.

Krankheiten und pathologische Zustände, die mit einem niedrigen cGMP-Spiegel verbunden sind oder bei denen eine Erhöhung des cGMP-Spiegels angestrebt wird und zu deren Therapie und Prophylaxe Verbindungen der Formel I eingesetzt werden können, sind zum Beispiel Herz-Kreislauf-Erkrankungen wie endotheliale Dysfunktion, diastolische Dysfunktion, Atherosklerose, Bluthochdruck, stabile und instabile Angina pectoris, Thrombosen, Restenosen, Myocardinfarkt, Schlaganfälle, Herzinsuffizienz oder Pulmonalhypertonie, oder zum Beispiel erektile Dysfunktion, Asthma bronchiale, chronische Niereninsuffizienz und Diabetes. Verbindungen der Formel I können darüber hinaus eingesetzt werden bei der Therapie der Leberzirrhose sowie zur Verbesserung einer eingeschränkten Lernfähigkeit oder Gedächtnisleistung.

Die Verbindungen der Formel I und ihre physiologisch verträglichen Salze können somit am Tier, bevorzugt am Säugetier, und insbesondere am Menschen als Arzneimittel für sich allein, in Mischungen untereinander oder in Form von pharmazeutischen Zubereitungen verwendet werden. Gegenstand der vorliegenden Erfindung sind daher auch die Verbindungen der Formel I und ihre physiologisch verträglichen Salze zur Anwendung als Arzneimittel, ihre Verwendung zur Normalisierung eines gestörten cGMP-Haushalts und insbesondere ihre Verwendung in der Therapie und Prophylaxe der oben genannten Krankheitsbilder, sowie ihre Verwendung zur Herstellung von Medikamenten dafür. Weiterhin sind Gegenstand der vorliegenden Erfindung pharmazeutische Präparate, die als aktiven Bestandteil eine wirksame Dosis mindestens einer Verbindung der Formel I und/oder eines physiologisch verträglichen Salzes und einen üblichen pharmazeutisch einwandfreien Träger enthalten. Gegenstand der vorliegenden Erfindung sind auch die als solche bereits bekannten Verbindungen der Formel I, in der R¹ für unsubstituiertes Phenyl steht, R², R³, R⁴, R⁵ und R⁷ für Wasserstoff stehen und R⁶ für unsubstituiertes Phenyl oder für 3,4-Dimethoxyphenyl steht, als Aktivatoren der löslichen Guanylatcyclase. Alle vorstehenden und nachfolgenden Ausführungen zur pharmakologischen Wirkung und zur Verwendung der Verbindungen der Formel I gelten für diese beiden Verbindungen entsprechend, Gegenstand der Erfindung sind also beispielsweise auch diese Verbindungen in allen ihren stereoisomeren Formen und Mischungen davon in allen Verhältnissen sowie ihre physiologisch verträglichen Salze zur Anwendung als Arzneimittel und pharmazeutische Präparate, die als aktiven Bestandteil eine wirksame Dosis mindestens einer dieser Verbindungen und/oder eines physiologisch verträglichen Salzes und einen üblichen pharmazeutisch einwandfreien Träger enthalten.

Die Arzneimittel können oral, zum Beispiel in Form von Pillen, Tabletten, Filmtabletten, Dragees, Granulaten, Hart- und Weichgelatinekapseln, wäßrigen, alkoholischen oder öligen Lösungen, Sirupen, Emulsionen oder Suspensionen, oder rektal, zum Beispiel in Form von Suppositorien, verabreicht werden. Die Verabreichung kann aber auch parenteral erfolgen, zum Beispiel subkutan, intramuskulär oder intravenös in Form von Injektionslösungen oder Infusionslösungen. Weitere in Betracht kommende Applikationsformen sind zum Beispiel die perkutane oder topische Applikation, zum Beispiel in Form von Salben, Tinkturen, Sprays oder transdermalen therapeutischen Systemen, oder die inhalative Applikation in Form von Nasalsprays oder Aerosolmischungen, oder zum Beispiel Mikrokapseln, Implantate oder Rods. Die bevorzugte Applikationsform hängt zum Beispiel von der zu behandelnden Krankheit und ihrer Stärke ab.

Die pharmazeutischen Präparate enthalten normalerweise 0.5 bis 90 Gewichtsprozent der Verbindungen der Formel I und/oder ihrer physiologisch verträglichen Salze. Die Herstellung der pharmazeutischen Präparate kann in an sich bekannter Weise erfolgen. Dazu werden ein oder mehrere Verbindungen der Formel I und/oder ihre physiologisch verträglichen Salze zusammen mit einem oder mehreren festen oder flüssigen galenischen Trägerstoffen und/oder Hilfsstoffen und, wenn gewünscht, in Kombination mit anderen Arzneimittelwirkstoffen mit therapeutischer oder prophylaktischer Wirkung in eine geeignete Verabreichungsform bzw. Dosierungsform gebracht, die dann als Arzneimittel in der Humanmedizin oder Veterinärmedizin verwendet werden kann. Pharmazeutische Präparate enthalten normalerweise 0.2 bis 500 mg, vorzugsweise 1 bis 200 mg, Wirkstoff der Formel I und/oder dessen physiologisch verträgliche Salze pro Dosis.

Für die Herstellung beispielsweise von Pillen, Tabletten, Dragees und Hartgelatinekapseln kann man Lactose, Stärke, zum Beispiel Maisstärke, oder Stärkederivate, Talk, Stearinsäure oder deren Salze, etc. verwenden. Trägerstoffe für Weichgelatinekapseln und Suppositorien sind zum Beispiel Fette, Wachse, halbfeste und flüssige Polyole, natürliche oder gehärtete Öle etc. Als Trägerstoffe für die Herstellung von Lösungen, zum Beispiel Injektionslösungen, oder von Emulsionen oder Sirupen eignen sich beispielsweise Wasser, physiologische Kochsalzlösung, Alkohole wie Ethanol, Glycerin, Polyole, Saccharose, Invertzucker, Glucose, Mannit, pflanzliche Öle etc. Die Verbindungen der Formel I und ihre physiologisch verträglichen Salze können auch lyophilisiert werden und die erhaltenen Lyophilisate zum Beispiel zur Herstellung von Injektionspräparaten oder Infusionspräparaten verwendet werden. Als Trägerstoffe für Mikrokapseln, Implantate oder Rods eignen sich zum Beispiel Mischpolymerisate aus Glykolsäure und Milchsäure.

Die erfindungsgemäßen pharmazeutischen Präparate können neben den Wirkstoffen und Trägerstoffen noch übliche Zusatzstoffe enthalten, zum Beispiel Füllstoffe, Spreng-, Binde-, Gleit-, Netz-, Stabilisierungs-, Emulgier-, Dispergier-, Konservierungs-, Süß-, Färbe-, Geschmacks- oder Aromatisierungs-, Dickungs-, Verdünnungsmittel, Puffersubstanzen, ferner Lösungsmittel oder Lösungsvermittler oder Mittel zur Erzielung eines Depoteffekts, Salze zur Veränderung des osmotischen Drucks, Überzugsmittel oder Antioxidantien.

Die Dosierung des zu verabreichenden Wirkstoffs der Formel I und/oder eines physiologisch verträglichen Salze davon hängt vom Einzelfall ab und ist wie üblich für eine optimale Wirkung den individuellen Gegebenheiten anzupassen. So hängt sie ab von der Art und Stärke der zu behandelnden Krankheit sowie von Geschlecht, Alter, Gewicht und individueller Ansprechbarkeit des zu behandelnden Menschen oder Tieres, von der Wirkstärke und Wirkdauer der eingesetzten Verbindungen, davon, ob akut oder chronisch therapiert wird oder Prophylaxe betrieben wird, oder davon, ob neben Verbindungen der Formel I weitere Wirkstoffe verabreicht werden. Im allgemeinen ist eine Tagesdosis von etwa 0.01 bis 100 mg/kg, vorzugsweise 0.1 bis 10 mg/kg, insbesondere 0.3 bis 5 mg/kg (jeweils mg pro kg Körpergewicht) bei Verabreichung an einen ca. 75 kg schweren Erwachsenen zur Erzielung der angestrebten Wirkung angemessen. Die Tagesdosis kann in einer Einzeldosis verabreicht werden oder, insbesondere bei der Applikation größerer Mengen, in mehrere, zum Beispiel zwei, drei oder vier Einzeldosen aufgeteilt werden. Gegebenenfalls kann es, je nach individuellem Verhalten, erforderlich werden, von der angegebenen Tagesdosis nach oben oder nach unten abzuweichen.

Die Verbindungen der Formel I aktivieren die lösliche Guanylatcyclase. Aufgrund dieser Eigenschaft können sie außer als Arzneimittelwirkstoffe in der Humanmedizin und Veterinärmedizin auch als wissenschaftliches Tool oder als Hilfsmittel für biochemische Untersuchungen eingesetzt werden, bei denen eine derartige Beeinflussung der Guanylatcyclase beabsichtigt ist, sowie für diagnostische Zwecke, zum Beispiel in der in vitro-Diagnostik von Zellproben oder Gewebsproben. Ferner können die Verbindungen der Formel I und ihre Salze, wie bereits oben erwähnt, als Zwischenprodukte in der Synthese weiterer Verbindungen dienen, insbesondere zur Herstellung weiterer Arzneimittelwirkstoffe.

Die nachfolgenden Beispiele erläutern die Erfindung, ohne sie einzuschränken.

### Beispiel 1

### 2-(2-(2-Fluorphenyl)ethyl)-3-hydroxy-3-phenyl-2,3-dihydro-isoindol-1-on

0.31 g (2.2 mmol) 2-(2-Fluorphenyl)ethylamin und 0.22 g (2.2 mmol) Triethylamin in 10 ml absolutem Toluol wurden bei Raumtemperatur tropfenweise mit einer Lösung von 0.49 g (2.0 mmol) 2-Benzoylbenzoesäurechlorid in 10 ml absolutem Toluol versetzt. Das Reaktionsgemisch wurde 3 h unter Rückfluß erhitzt. Nach Abkühlen wurde die trübe Lösung mit 20 ml Wasser versetzt. Die organische Phase wurde abgetrennt, mit 20 ml Toluol verdünnt und dreimal mit 1 M Salzsäure gewaschen. Die vereinigten wäßrigen Phasen wurden zur Vervollständigung der Extraktion mit Diethylether extrahiert. Die vereinigten organischen Phasen wurden dreimal mit 1 M Natronlauge und einmal mit gesättigter Natriumchloridlösung gewaschen, über Natriumsulfat getrocknet und im Rotationsverdampfer zur Trockne eingeengt. Der Rückstand wurde aus Toluol umkristallisiert. Das erhaltene farblose kristalline Produkt wurde abgesaugt und getrocknet. Ausbeute: 0.53 g. Schmp.: 208 °C.

Die folgenden, in der Tabelle 1 aufgeführten Verbindungen der Formel Ib wurden analog Beispiel 1 unter Verwendung des entsprechenden Amins hergestellt.

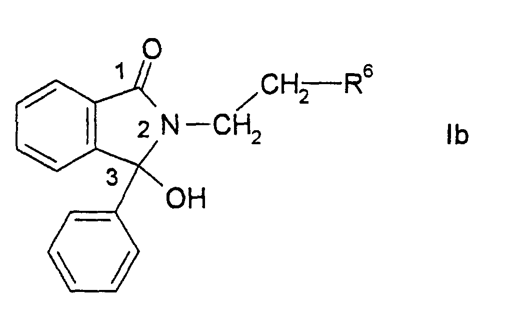

**Tabelle 1:**

| Beispielverbindungen der Formel Ib | | | |
|---|---|---|---|
| Beispiel | R⁶ | Ausbeute (%) | Schmp. (°C) |
| 2 | 2,4-Dichlorphenyl | 65 | 196 |
| 3 | 2-Methoxyphenyl | 82 | 151 |
| 4 | 3-Fluorphenyl | 72 | 158 |
| 5 | 4-Fluorphenyl | 90 | 156 |
| 6 | 3-Chlorphenyl | 40 | 110 |
| 7 | 4-Methylphenyl | 100 | 144 |
| 8 | 4-Phenoxyphenyl | 76 | 144 |
| 9 | 3,4-Dichlorphenyl | 51 | 150 |
| 10 | 2,3-Dimethoxyphenyl | 79 | 171 |
| 11 | 3-Methoxyphenyl | 82 | 118 |
| 12 | 2-Chlorphenyl | 87 | 201 |
| 13 | 3-Trifluormethylphenyl | 73 | 110 |
| 14 | 4-Chlorphenyl | 70 | 165 |

### Beispiel 15

### 2-(2-(2,2-Diphenylethyl)-3-hydroxy-3-phenyl-2,3-dihydro-isoindol-1-on

Die Verbindung wurde analog Beispiel 1 unter Verwendung von 2,2-Diphenylethylamin hergestellt. Ausbeute: 32 %. Schmp.: 230 °C.

### Beispiel 16

### 3-Hydroxy-2-(2-(4-hydroxyphenyl)ethyl)-3-phenyl-2,3-dihydro-isoindol-1-on

1.46 g (10.63 mmol) 2-(4-Hydroxyphenyl)ethylamin und 1.08 g (10.63 mmol) Triethylamin in 30 ml absolutem Toluol wurden bei Raumtemperatur tropfenweise mit einer Lösung von 2.0 g (8.17 mmol) 2-Benzoylbenzoesäurechlorid in 20 ml absolutem Toluol versetzt. Das Reaktionsgemisch wurde 3 h unter Rückfluß erhitzt, abgekühlt und die trübe Lösung mit 20 ml Wasser versetzt. Die organische Phase wurde abgetrennt, dreimal mit 1 M wäßriger Salzsäure gewaschen und im Rotationsverdampfer zur Trockne eingeengt. Der feste Rückstand wurde durch Säulenchromatographie an Kieselgel 60 gereinigt. Es wurde mit einem Toluol-Essigester-Gradienten (0 bis 20 % Essigester) eluiert. Die produkthaltigen Fraktionen wurden im Rotationsverdampfer zur Trockne eingeengt. Der Rückstand wurde in Diethylether suspendiert und abgesaugt. Ausbeute: 0.21 g. Schmp.: 188 °C.

### Beispiel 17

### 3-(4-Fluorphenyl)-3-hydroxy-2-(2-(3-methoxyphenyl)ethyl)-2,3-dihydro-isoindol-1-on

2.0 g (7.11 mmol) N-(2-(3-Methoxyphenyl)ethyl)phthalimid in 30 ml absolutem Tetrahydrofuran wurden bei 0 °C unter Schutzgas mit 5.37 g (14.22 mmol) einer 1 M Lösung von 4-Fluorphenylmagesiumbromid in Diethylether versetzt. Man ließ das Reaktionsgemisch sich auf Raumtemperatur erwärmen und versetzte nach 3 h mit 20 ml gesättigter Natriumhydrogencarbonatlösung. Die wäßrige Phase wurde zweimal mit Diethylether extrahiert. Die vereinigten organischen Phasen wurden getrocknet und im Rotationsverdampfer zur Trockne eingeengt. Der feste Rückstand wurde aus Toluol umkristallisiert. Es wurden farblose Nadeln erhalten. Ausbeute: 2.21 g. Schmp.: 134 °C.

Die folgenden, in der Tabelle 2 aufgeführten Verbindungen der Formel Ic wurden analog Beispiel 17 unter Verwendung des entsprechenden Phthalimids und des entsprechenden Grignard-Reagenzes hergestellt. Die Reinigung erfolgte wie in der Tabelle 2 angegeben.

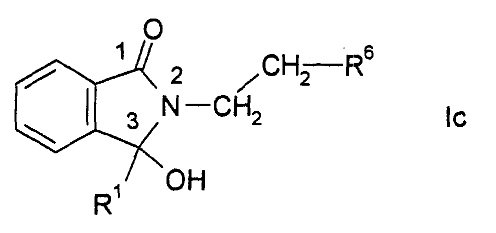

**Tabelle 2:**

| Beispielverbindungen der Formel Ic | | | | | |
|---|---|---|---|---|---|
| Beispiel | R⁶ | R¹ | Reinigung (Lösungsmittel) | Ausb. (%) | Schmp. (°C) |
| 18 | 3-Hydroxyphenyl | Phenyl | Chromatographie | 52 | 149 |
| 19 | 2-Chlorphenyl | 4-Methylphenyl | Kristallisation (1) | 71 | 182 |
| 20 | 2-Chlorphenyl | Cyclopentyl | aufgeschäumt (2) | 51 | 52 |
| 21 | 3-Methoxyphenyl | 3-Methylphenyl | Chromatographie | 88 | Öl |
| 22 | 3-Methoxyphenyl | 2-Methylphenyl | Chromatographie, Kristallisation (2) | 23 | 128 |
| 23 | 3-Methoxyphenyl | 4-Methylphenyl | Chromatographie, Kristallisation (3) | 53 | 84 |
| 24 | 3-Methoxyphenyl | 4-Chlorphenyl | Chromatographie, Kristallisation (3) | 60 | 106 |
| 25 | 3-Methoxyphenyl | Cyclohexyl | Chromatographie | 87 | Öl |
| 26 | 3-Methoxyphenyl | Cyclopentyl | Chromatographie, Kristallisation (4) | 47 | 102 |

Bei der Angabe der Lösungsmittel in Tabelle 2 bedeuten:
(1): aus Diethylether/tert-Butylmethylether; (2): aus Diethylether; (3): aus Diisopropylether; (4): aus n-Heptan/Diethylether

### Beispiel 27

### 3-Hydroxy-3-(4-methoxyphenyl)-2-(2-(3-methoxyphenyl)ethyl)-2,3-dihydro-isoindol-1-on

104 mg (4.28 mmol) Magnesiumspäne in 10 ml absolutem Diethylether wurden mit 0.79 g (4.27 mmol) 4-Bromanisol versetzt. Durch Zugabe eines Tropfens Brom wurde die Reaktion gestartet. Nach ca. 2 h war das Magnesium vollständig in Lösung gegangen. Nach Abkühlen auf 0 °C wurden 0.3 g (1.07 mmol) N-(2-(3-Methoxyphenyl)ethyl)phthalimid zugegeben. Man ließ das Reaktionsgemisch sich auf Raumtemperatur erwärmen und versetzte nach 2 h mit 5 ml gesättigter Natriumhydrogencarbonatlösung. Die wäßrige Phase wurde zweimal mit Diethylether extrahiert. Die vereinigten organischen Phasen wurden getrocknet und im Rotationsverdampfer zur Trockne eingeengt. Das erhaltene Öl wurde durch Chromatographie gereinigt. Es wurde mit einem Dichlormethan-Methanol-Gradienten (0 bis 2 % Methanol) eluiert. Das als farbloses Öl angefallene Produkt wurde durch Ko-Evaporieren mit Diethylether aufgeschäumt. Ausbeute: 0.32 g. Schmp.: 119 °C.

### Beispiel 28

### 3-Hydroxy-2-(2-(3-methoxyphenyl)ethyl)-3-(4-trifluormethylphenyl)-2,3-dihydro-isoindol-1-on

172 mg (7.08 mmol) Magnesiumspäne in 10 ml absolutem Diethylether wurden bei Raumtemperatur mit 1.6 g (7.11 mmol) 4-Trifluormethylbrombenzol versetzt. Durch Zugabe eines Tropfens Brom wurde die Reaktion gestartet. Nach ca. 2 h war das Magnesium vollständig in Lösung gegangen. Nach Abkühlen auf 0 °C wurden 0.5 g (1.78 mmol) N-(2-(3-Methoxyphenyl)ethyl)phthalimid zugegeben. Man ließ das Reaktionsgemisch sich auf Raumtemperatur erwärmen und versetzte nach 2 h mit 5 ml gesättigter Natriumhydrogencarbonatlösung. Die wäßrige Phase wurde zweimal mit Diethylether extrahiert. Die vereinigten organischen Phasen wurden getrocknet und im Rotationsverdampfer zur Trockne eingeengt. Der rote kristalline Rückstand wurde aus Toluol umkristallisiert. Es wurde ein farbloses Produkt erhalten. Ausbeute: 0.49 g. Schmp.: 167 °C.

### Beispiel 29

### 3-Hydroxy-2-(2-(3-methoxyphenyl)ethyl)-3-(4-pyridyl)-2,3-dihydro-isoindol-1-on

0.69 g (3.55 mmol) 4-Brompyridin-Hydrochlorid wurden zunächst in die freie Base überführt. Dazu wurde das Salz in 5 ml 1 M Natronlauge gelöst, die Lösung dreimal mit Diethylether extrahiert, die vereinigten organischen Phasen getrocknet und im Rotationsverdampfer zur Trockne eingeengt. Der Rückstand wurde in 5 ml absolutem Diethylether gelöst und die Lösung bei -70 °C tropfenweise zu einer Mischung von 1.6 M Butyllithium-Lösung in n-Hexan (3.98 mmol) und 10 ml absolutem Diethylether hinzugefügt. Es wurde 30 min bei -70 °C gerührt und dann eine Lösung von 0.5 g (1.78 mmol) N-(2-(3-Methoxyphenyl)ethyl)phthalimid in 10 ml absolutem Tetrahydrofuran zugetropft (Farbumschlag von gelb nach braun). Nach 1 h ließ man das Reaktionsgemisch sich auf Raumtemperatur erwärmen, versetzte mit 10 ml gesättigter Ammoniumchloridlösung und extrahierte dreimal mit Diethylether. Die vereinigten organischen Phasen wurden einmal mit gesättigter Natriumchloridlösung gewaschen, getrocknet und im Rotationsverdampfer zur Trockne eingeengt. Durch Umkristallisation des kristallinen Rückstandes aus Diisopropylether/Ethanol wurde ein farbloses kristallines Produkt erhalten.
Ausbeute: 0.28 g. Schmp.: 190 °C.

### Beispiel 30

### 3-Hydroxy-2-(2-(3-methoxyphenyl)ethyl)-3-(3-pyridyl)-2,3-dihydro-isoindol-1-on

Eine 1.6 M Butyllithium-Lösung (3.99 mmol) in absolutem Diethylether wurde bei -70 °C tropfenweise mit einer Lösung von 0.56 g (3.56 mmol) 3-Brompyridin in absolutem Diethylether versetzt. Es trat eine Gelbfärbung auf und es bildete sich ein Niederschlag. Nach 1 h bei -70 °C wurde eine Lösung von 0.5 g (1.78 mmol) N-(2-(3-Methoxyphenyl)ethyl)phthalimid in 10 ml absolutem Tetrahydrofuran zugetropft. Nach Erwärmen auf Raumtemperatur wurde das Reaktionsgemisch noch 1 h nachgerührt und dann mit 10 ml gesättigter Ammoniumchloridlösung versetzt. Es wurde dreimal mit Diethylether extrahiert.

Die vereinigten organischen Phasen wurden einmal mit gesättigter Natriumchloridlösung gewaschen, getrocknet und im Rotationsverdampfer zur Trockne eingeengt. Das farblose ölige Rohprodukt wurde durch Chromatographie gereinigt. Es wurde mit einem Dichlormethan-Methanol-Gradienten (0 bis 3 % Methanol) eluiert. Das erhaltene farblose Öl wurde beim Stehenlassen bei Raumtemperatur fest. Ausbeute: 0.34 g. Schmp.: 97 °C.

### Beispiel 31

### 3-Hydroxy-2-(2-(3-methoxyphenyl)ethyl)-3-(2-thienyl)-2,3-dihydro-isoindol-1-on

0.3 g (1.07 mmol) N-(2-(3-Methoxyphenyl)ethyl)phthalimid wurden in 10 ml absolutem Tetrahydrofuran gelöst und bei 0 °C tropfenweise mit einer 1 M Lösung von 0.14 g (1.6 mmol) 2-Thienyllithium versetzt. Nachdem das Reaktionsgemisch auf Raumtemperatur gekommen war, wurde noch 2 h nachgerührt und dann mit 10 ml gesättigter Ammoniumchloridlösung versetzt. Es wurde dreimal mit Diethylether extrahiert und die vereinigten organischen Phasen wurden einmal mit gesättigter Natriumchloridlösung gewaschen, getrocknet und im Rotationsverdampfer zur Trockne eingeengt. Das ölige Rohprodukt wurde durch Chromatographie gereinigt. Es wurde mit einem Dichlormethan-Methanol-Gradienten (0 bis 1 % Methanol) eluiert. Ausbeute: 0.2 g eines Öls.

### Beispiel 32

### 5,6-Dichlor-3-(4-fluorphenyl)-3-hydroxy-2-(2-(3-methoxyphenyl)ethyl)-2,3-dihydro-isoindol-1 -on

0.4 g (1.14 mmol) 4,5-Dichlor-N-(3-methoxyphenyl)ethyl)phthalimid wurden in 10 ml absolutem Tetrahydrofuran gelöst und bei 0 °C mit einer 1 M Lösung von 4-Fluorphenylmagnesiumbromid (1.71 mmol) in Tetrahydrofuran versetzt. Nach 3 h Rühren bei Raumtemperatur wurde nochmals eine 1 M Lösung von 4-Fluorphenylmagnesiumbromid (1 mmol) in Tetrahydrofuran zugegeben. Nach 1 h wurde das Reaktionsgemisch mit 10 ml gesättigter Ammoniumchloridlösung versetzt. Die organische Phase wurde abgetrennt und die wäßrige Phase dreimal mit Diethylether extrahiert. Die vereinigten organischen Phasen wurden mit gesättigter Natriumchloridlösung gewaschen, getrocknet und im Rotationsverdampfer zur Trockne eingeengt. Der farblose Rückstand wurde in Diethylether suspendiert und abgesaugt. Ausbeute: 0.13 g. Schmp.: 169 °C.

### Beispiel 33

### 4,5,6,7-Tetrafluor-3-(4-fluorphenyl)-3-hydroxy-2-(2-(3-methoxyphenyl)ethyl)-2,3-dihydro-isoindol-1 -on

0.4 g (1.13 mmol) 3,4,5,6-Tetrafluor-N-(3-methoxyphenyl)ethyl)phthalimid wurden in 10 ml absolutem Tetrahydrofuran gelöst und bei 0 °C mit einer 1 M Lösung von 4-Fluorphenylmagnesiumbromid (2.3 mmol) in Tetrahydrofuran versetzt. Nach 3 h Rühren bei Raumtemperatur wurde das Reaktionsgemisch mit 10 ml gesättigter Ammoniumchloridlösung versetzt, die organische Phase abgetrennt und die wäßrige Phase dreimal mit Diethylether extrahiert. Die vereinigten organischen Phasen wurden mit gesättigter Natriumchloridlösung gewaschen, getrocknet und im Rotationsverdampfer zur Trockne eingeengt. Der farblose Rückstand wurde aus Toluol umkristallisiert. Ausbeute: 0.32 g. Schmp.: 161 °C.

### Beispiel 34

### 4,7-Difluor-3-(4-fluorphenyl)-3-hydroxy-2-(2-(3-methoxyphenyl)ethyl)-2, 3-dihydro-isoindol-1-on

0.25 g (0.79 mmol) 3,6-Difluor-N-(3-methoxyphenyl)ethyl)phthalimid wurden in 10 ml absolutem Tetrahydrofuran gelöst und bei 0 °C mit einer 1 M Lösung von 0.31 g (1.58 mmol) 4-Fluorphenylmagnesiumbromid in Tetrahydrofuran versetzt. Nach 3 h Rühren bei Raumtemperatur wurde das Reaktionsgemisch mit 10 ml gesättigter Ammoniumchloridlösung versetzt, die organische Phase abgetrennt und die wäßrige Phase dreimal mit Diethylether extrahiert. Die vereinigten organischen Phasen wurden mit gesättigter Natriumchloridlösung gewaschen, getrocknet und im Rotationsverdampfer zur Trockne eingeengt. Der farblose Rückstand wurde in Diethylether suspendiert und abgesaugt. Ausbeute: 0.19 g. Schmp.: 152°C

### Beispiel 35

### 4,7-Difluor-3-hydroxy-2-(2-(3-methoxyphenyl)ethyl)-3-phenyl-2,3-dihydroisoindol-1-on

0.17 g (0.53 mmol) 3,6-Difluor-N-(3-methoxyphenyl)ethyl)phthalimid wurden in 5 ml absolutem Tetrahydrofuran gelöst und bei 0 °C mit einer 1 M Lösung von 0.19 g (1.07 mmol) Phenylmagnesiumbromid in Tetrahydrofuran versetzt. Nach 3 h Rühren bei Raumtemperatur wurde das Reaktionsgemisch mit 5 ml gesättigter Ammoniumchloridlösung versetzt, die organische Phase abgetrennt und die wäßrige Phase dreimal mit Diethylether extrahiert. Die vereinigten organischen Phasen wurden mit gesättigter Natriumchloridlösung gewaschen, getrocknet und im Rotationsverdampfer zur Trockne eingeengt. Der farblose Rückstand wurde in Diethylether suspendiert und abgesaugt. Ausbeute: 0.085 g. Schmp.: 141 °C.

### Beispiel 36

### 3-(4-Fluorphenyl)-3-hydroxy-2-(2-(3-methoxyphenyl)ethyl)-4,5,6,7-tetramethyl-2,3-dihydro-isoindol-1-on

0.23 g (0.68 mmol) N-(3-Methoxyphenyl)ethyl)-3,4,5,6-tetramethylphthalimid wurden in 5 ml absolutem Tetrahydrofuran gelöst und bei 0 °C mit einer 1 M Lösung von 0.27 g (1.36 mmol) 4-Fluorphenylmagnesiumbromid in Tetrahydrofuran versetzt. Nach 3 h Rühren bei Raumtemperatur wurde nochmals eine 1 M Lösung von 4-Fluorphenylmagnesiumbromid (2 mmol) in Tetrahydrofuran zugegeben. Nach 1 h Rühren bei Raumtemperatur wurde das Reaktionsgemisch dann mit 5 ml gesättigter Ammoniumchloridlösung versetzt. Die organische Phase wurde abgetrennt und die wäßrige Phase dreimal mit Diethylether extrahiert. Die vereinigten organischen Phasen wurden mit gesättigter Natriumchloridlösung gewaschen, getrocknet und im Rotationsverdampfer zur Trockne eingeengt. Der ölige Rückstand wurde aus Diisopropylether umkristallisiert. Ausbeute: 0.18 g. Schmp.: 181 °C.

### Pharmakologische Untersuchungen

### Aktivierung der löslichen Guanylatcyclase

Die Aktivierung der löslichen Guanylatcyclase (sGC), die die Umwandlung von Guanosintriphosphat (GTP) in cyclisches Guanosinmonophosphat (cGMP) und Pyrophosphat katalysiert, durch die erfindungsgemäßen Verbindungen wurde mit Hilfe eines Enyzm-lmmuno-Assays (EIA) der Firma Amersham quantifiziert. Dazu wurden die Prüfsubstanzen zunächst mit sGC in Mikrotiterplatten inkubiert und dann die Menge des entstandenen cGMP bestimmt.

Die eingesetzte sGC war aus Rinderlunge isoliert worden (siehe Methods in Enzymology, Band 195, S. 377). Die Testlösungen (100 µl pro well) enthielten 50 mM Triethanolamin(TEA)-Puffer (pH 7.5), 3 mM MgCl₂, 3 mM reduziertes Glutathion (GSH), 0.1 mM GTP, 1 mM 3-Isobutyl-1-methylxanthin (IBMX), geeignet verdünnte Enzymlösung sowie die Prüfsubstanz bzw. bei den Kontrollversuchen Lösungsmittel. Die Prüfsubstanzen wurden in Dimethylsulfoxid (DMSO) gelöst und die Lösung mit DMSO/Wasser verdünnt, so daß die Endkonzentration an Prüfsubstanz im Testansatz 50 µM betrug. Die DMSO-Konzentration im Testansatz betrug 5 % (v/v). Die Reaktion wurde durch Zugabe der sGC gestartet. Der Reaktionsmix wurde für 15 bis 20 Minuten bei 37 °C inkubiert und dann die Reaktion durch Eiskühlung und Zugabe des Stop-Reagenz (50 mM EDTA, pH 8.0) gestoppt. Ein Aliquot von 50 µl wurde entnommen und zur Bestimmung des cGMP-Gehaltes mit dem Acetylierungs-Protokoll des Amersham-cGMP-EIA-Kits eingesetzt. Die Absorption der Proben wurde bei 450 nm (Referenz Wellenlänge 620 nm) in einem Mikrotiterplatten-Lesegerät gemessen. Die cGMP-Konzentration wurde über eine Eichkurve ermittelt, die unter denselben Versuchsbedingungen erhalten wurde. Die Aktivierung der sGC durch eine Prüfsubstanz wird angegeben als n-fache Stimulation der basalen Enzymaktivität, die bei den Kontroliversuchen (mit Lösungsmittel statt Prüfsubstanz) gefunden wurde (berechnet nach der Formel n-fache Stimulierung = [cGMP]_{Prüfsubstanz} *l* [cGMP]_{Kontrolle}).

Es wurden folgende Werte ermittelt:

| Verbindung | Konzentration | n-fache Stimulierung |
|---|---|---|
| Beispiel 11 | 100 µM | 23.5 fach |
| Beispiel 17 | 50 µM | 16 fach |
| Beispiel 20 | 50 µM | 8.2 fach |
| Beispiel 29 | 50 µM | 11.8 fach |
| Beispiel 33 | 50 µM | 9.5 fach |
| A (Vergleich) | 50 µM | 2 fach |

Die zum Vergleich untersuchte Verbindung A ist das 1-Benzyl-3-(5-hydroxymethyl-2-furyl)-indazol (C.-C. Wu et al., Brit. J. Pharmacol. 116 (1995) 1973).

## Patentansprüche

1. Verbindungen der Formel I, 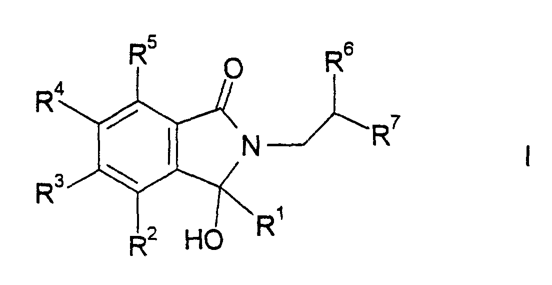 in der
R¹ für (C₃-C₇)-Cycloalkyl, Phenyl oder den Rest eines 5-gliedrigen oder 6-gliedrigen aromatischen Heterocyclus, der ein oder zwei gleiche oder verschiedene Heteroatome aus der Reihe N, O, S enthält, steht, wobei der Phenylrest und der heterocyclische Rest unsubstituiert sind oder durch einen oder mehrere gleiche oder verschiedene Reste aus der Reihe (C₁-C₄)-Alkyl, Halogen, Hydroxy, (C₁-C₄)-Alkoxy, Benzyloxy, Phenoxy, Benzyl, Phenyl, Trifluormethyl, Cyan, Hydroxycarbonyl, ((C₁-C₄)-Alkoxy)carbonyl, Aminocarbonyl, Nitro, Amino, (C₁-C₄)-Alkylamino, Di-((C₁-C₄)-alkyl)amino und ((C₁-C₄)-Alkyl)carbonylamino substituiert sind;
R², R³, R⁴ und R⁵ unabhängig voneinander für Wasserstoff, Halogen, (C₁-C₄)-Alkyl oder (C₁-C₄)-Alkoxy stehen;
R⁶ für Phenyl steht, das unsubstituiert ist oder durch einen oder mehrere gleiche oder verschiedene Reste aus der Reihe (C₁-C₄)-Alkyl, Halogen, Hydroxy, (C₁-C₄)-Alkoxy, Benzyloxy, Phenoxy, Benzyl, Phenyl, Trifluormethyl, Cyan, Hydroxycarbonyl, ((C₁-C₄)-Alkoxy)carbonyl, Aminocarbonyl, Nitro, Amino, (C₁-C₄)-Alkylamino, Di-((C₁-C₄)-alkyl)amino und ((C₁-C₄)-Alkyl)carbonylamino substituiert ist;
R⁷ unabhängig von R⁶ eine der Bedeutungen von R⁶ hat oder für Wasserstoff steht;
in allen ihren stereoisomeren Formen und Mischungen davon in allen Verhältnissen, und ihre physiologisch verträglichen Salze;
wobei, wenn R¹ für unsubstituiertes Phenyl steht und R², R³, R⁴, R⁵ und R⁷ für Wasserstoff stehen, dann nicht gleichzeitig R⁶ für unsubstituiertes Phenyl oder für 3,4-Dimethoxyphenyl stehen kann.

2. Verbindungen der Formel I gemäß Anspruch 1, in der R¹ für unsubstituiertes Phenyl oder Phenyl, das durch einen oder zwei gleiche oder verschiedene Reste aus der Reihe (C₁-C₄)-Alkyl und Halogen substituiert ist, steht, in allen ihren stereoisomeren Formen und Mischungen davon in allen Verhältnissen, und ihre physiologisch verträglichen Salze.

3. Verbindungen der Formel I gemäß den Ansprüchen 1 und/oder 2, in der R², R³, R⁴ und R⁵ unabhängig voneinander für Wasserstoff, Fluor, Chlor oder Methyl stehen, in allen ihren stereoisomeren Formen und Mischungen davon in allen Verhältnissen, und ihre physiologisch verträglichen Salze.

4. Verbindungen der Formel I gemäß einem oder mehreren der Ansprüche 1 bis 3, in der R⁶ für Phenyl steht, das unsubstituiert ist oder durch einen oder zwei gleiche oder verschiedene Reste aus der Reihe (C₁-C₄)-Alkyl, Halogen und (C₁-C₄)-Alkoxy substituiert ist, in allen ihren stereoisomeren Formen und Mischungen davon in allen Verhältnissen, und ihre physiologisch verträglichen Salze.

5. Verbindungen der Formel I gemäß einem oder mehreren der Ansprüche 1 bis 4, in der R⁶ für Phenyl steht, das durch einen oder zwei gleiche oder verschiedene Reste aus der Reihe Halogen und (C₁-C₄)-Alkoxy substituiert ist, in allen ihren stereoisomeren Formen und Mischungen davon in allen Verhältnissen, und ihre physiologisch verträglichen Salze.

6. Verbindungen der Formel I gemäß einem oder mehrerem der Anspüche 1 bis 5, in der R⁷ für Wasserstoff steht, in allen ihren stereoisomeren Formen und Mischungen davon in allen Verhältnissen, und ihre physiologisch verträglichen Salze.

7. Verbindungen der Formel I gemäß einem oder mehreren der Ansprüche 1 bis 6, in der
R¹ für unsubstituiertes Phenyl oder Fluorphenyl steht;
R², R³, R⁴ und R⁵ für Wasserstoff stehen;
R⁶ für Methoxyphenyl oder für Chlorphenyl steht;
R⁷ für Wasserstoff steht;
in allen ihren stereoisomeren Formen und Mischungen davon in allen Verhältnissen, und ihre physiologisch verträglichen Salze.

8. Verfahren zur Herstellung von Verbindungen der Formel I gemäß einem oder mehreren der Ansprüche 1 bis 7, **dadurch gekennzeichnet, daß** man eine Benzoesäure der Formel II oder ein aktiviertes Derivat davon mit einem Amin der Formel III umsetzt, oder daß man ein Phthalimid der Formel IV mit einer metallorganischen Verbindung der Formel V umsetzt, 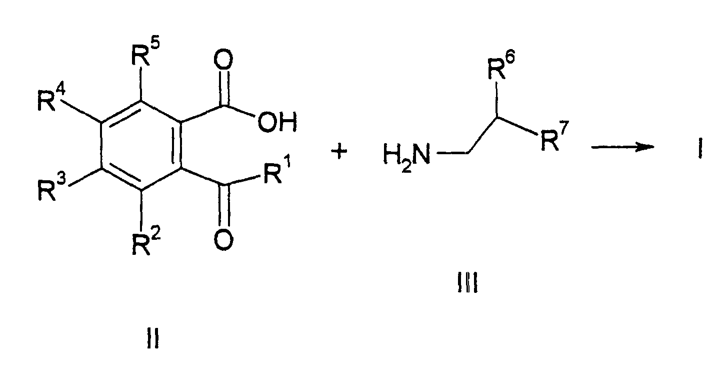 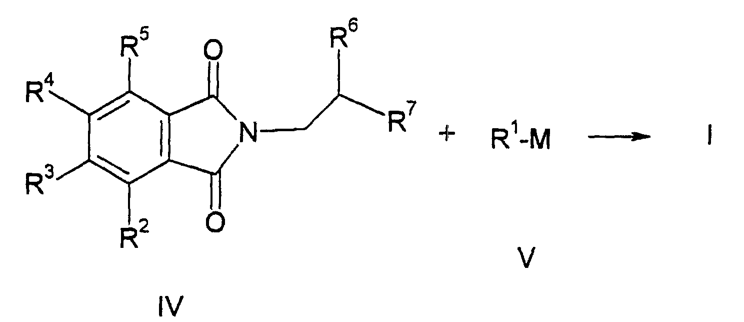 wobei in den Formeln II, III, IV und V die Reste R¹, R², R³, R⁴, R⁵, R⁶ und R⁷ die in den Ansprüchen 1 bis 7 angegebenen Bedeutungen haben und M für Li oder MgCI, MgBr oder Mgl steht.

9. Verbindungen der Formel I gemäß einem oder mehreren der Ansprüche 1 bis 7 und/oder ihre physiologisch verträglichen Salze zur Anwendung als Arzneimittel.

10. Pharmazeutisches Präparat, **dadurch gekennzeichnet, daß** es eine oder mehrere Verbindungen der Formel I gemäß einem oder mehreren der Ansprüche 1 bis 7 und/oder ihre physiologisch verträglichen Salze und einen pharmazeutisch einwandfreien Träger enthält.

11. Verwendung von Verbindungen der Formel I gemäß einem oder mehreren der Ansprüche 1 bis 7 und/oder ihren physiologisch verträglichen Salzen zur Herstellung eines Medikaments zur Aktivierung der löslichen Guanylatcyclase.

12. Verwendung von Verbindungen der Formel I gemäß einem oder mehreren der Ansprüche 1 bis 7 und/oder ihren physiologisch verträglichen Salzen zur Herstellung eines Medikaments zur Therapie oder Prophylaxe von Herz-Kreislauf-Erkrankungen, endothelialer Dysfunktion, diastolischer Dysfunktion, Atherosklerose, Bluthochdruck, Angina pectoris, Thrombosen, Restenosen, Myocardinfarkt, Schlaganfällen, Herzinsuffizienz, Pulmonalhypertonie, erektiler Dysfunktion, Asthma bronchiale, chronischer Niereninsuffizienz, Diabetes oder Leberzirrhose oder zur Verbesserung einer eingeschränkten Lernfähigkeit oder Gedächtnisleistung.

## Claims

1. A compound of the formula I 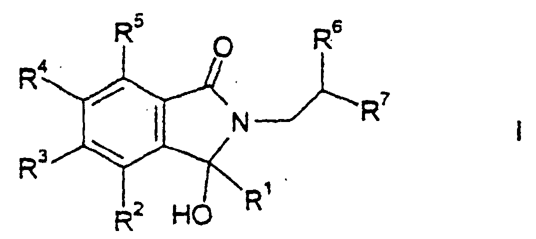 in which
R¹ represents (C₃-C₇)-cycloalkyl, phenyl or the radical of a 5-membered or 6-membered aromatic heterocycle which contains one or two identical or different heteroatoms selected from the group consisting of N, O and S, where the phenyl radical and the heterocyclic radical are unsubstituted or substituted by one or more identical or different radicals selected from the group consisting of (C₁-C₄)-alkyl, halogen, hydroxyl, (C₁-C₄)-alkoxy, benzyloxy, phenoxy, benzyl, phenyl, trifluoromethyl, cyano, hydroxycarbonyl, ((C₁-C₄)-alkoxy)carbonyl, aminocarbonyl, nitro, amino, (C₁-C₄)-alkylamino, di-((C₁-C₄)-alkyl)amino and ((C₁-C₄)-alkyl)carbonylamino;
R², R³, R⁴ and R⁵ independently of one another are hydrogen, halogen, (C₁-C₄)-alkyl or (C₁-C₄)-alkoxy;
R⁶ is phenyl which is unsubstituted or substituted by one or more identical or different radicals selected from the group consisting of (C₁-C₄)-alkyl, halogen, hydroxyl, (C₁-C₄)-alkoxy, benzyloxy, phenoxy, benzyl, phenyl, trifluoromethyl, cyano, hydroxycarbonyl, ((C₁-C₄)-alkoxy)carbonyl, aminocarbonyl, nitro, amino, (C₁-C₄)-alkylamino, di-((C₁-C₄)-alkyl)amino and ((C₁-C₄)-alkyl)carbonylamino;
R⁷ independently of R⁶ has one of the meanings of R⁶ or is hydrogen,
in all its stereoisomeric forms and mixtures thereof in all ratios, and its physiologically acceptable salts;
where, if R¹ is unsubstituted phenyl and R², R³, R⁴, R⁵ and R⁷ are hydrogen, R⁶ may not simultaneously be unsubstituted phenyl or 3,4-dimethoxyphenyl.

2. A compound of the formula I as claimed in claim 1 in which R¹ is unsubstituted phenyl or phenyl which is substituted by one or two identical or different radicals selected from the group consisting of (C₁-C₄)-alkyl and halogen, in all its stereoisomeric forms and mixtures thereof in all ratios, and its physiologically acceptable salts.

3. A compound of the formula I as claimed in claim 1 and/or 2 in which R², R³, R⁴ and R⁵ independently of one another are hydrogen, fluorine, chlorine or methyl, in all its stereoisomeric forms and mixtures thereof in all ratios, and its physiologically acceptable salts.

4. A compound of the formula I as claimed in one or more of claims 1 to 3 in which R⁶ is phenyl which is unsubstituted or substituted by one or two identical or different radicals selected from the group consisting of (C₁-C₄)-alkyl, halogen and (C₁-C₄)-alkoxy, in all its stereoisomeric forms and mixtures thereof in all ratios, and its physiologically acceptable salts.

5. A compound of the formula I as claimed in one or more of claims 1 to 4 in which R⁶ is phenyl which is substituted by one or two identical or different radicals selected from the group consisting of halogen and (C₁-C₄)-alkoxy, in all its stereoisomeric forms and mixtures thereof in all ratios, and its physiologically acceptable salts.

6. A compound of the formula I as claimed in one or more of claims 1 to 5 in which R⁷ is hydrogen, in all its stereoisomeric forms and mixtures thereof in all ratios, and its physiologically acceptable salts.

7. A compound of the formula I as claimed in one or more of claims 1 to 6 in which
R¹ is unsubstituted phenyl or fluorophenyl;
R², R³, R⁴ and R⁵ are hydrogen;
R⁶ is methoxyphenyl or chlorophenyl;
R⁷ is hydrogen,
in all its stereoisomeric forms and mixtures thereof in all ratios, and its physiologically acceptable salts.

8. A process for preparing compounds of the formula I as claimed in one or more of claims 1 to 7, which comprises reacting a benzoic acid of the formula II or an activated derivative thereof with an amine of the formula III, or reacting a phthalimide of the formula IV with an organometallic compound of the formula V 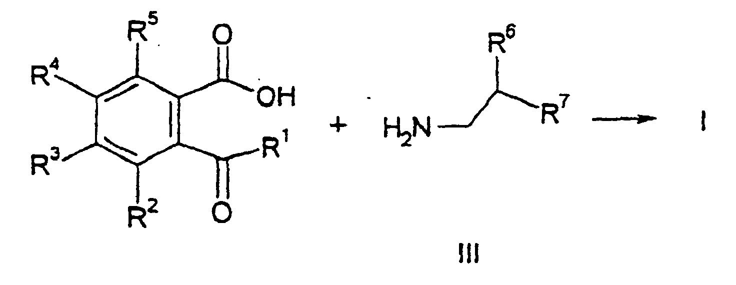 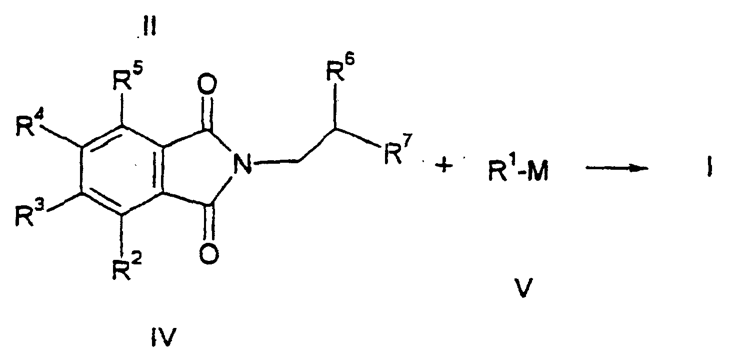 where in the formulae II, III, IV and V the radicals R¹, R², R³, R⁴, R⁵, R⁶ and R⁷ are as defined in claims 1 to 7 and M is Li or MgCl, MgBr or MgI.

9. A compound of the formula I as claimed in one or more of claims 1 to 7 and/or its physiologically acceptable salts for use as a pharmaceutical.

10. A pharmaceutical preparation, which comprises one or more compounds of the formula I as claimed in one or more of claims 1 to 7 and/or its physiologically acceptable salts and a pharmaceutically unobjectionable carrier.

11. The use of compounds of the formula I as claimed in one or more of claims 1 to 7 and/or their physiologically acceptable salts for preparing a medicament for the activation of soluble guanylate cyclase.

12. The use of compounds of the formula I as claimed in one or more of claims 1 to 7 and/or their physiologically acceptable salts for preparing a medicament for the therapy or prophylaxis of cardiovascular diseases, endothelial dysfunction, diastolic dysfunction, atherosclerosis, hypertension, angina pectoris, thromboses, restenoses, myocardial infarction, strokes, cardiac insufficiency, pulmonary hypertonia, erectile dysfunction, bronchial asthma, chronic kidney insufficiency, diabetes or cirrhosis of the liver or for improving a restricted ability to leam or memory performance.

## Revendications

1. Composés de formule I, 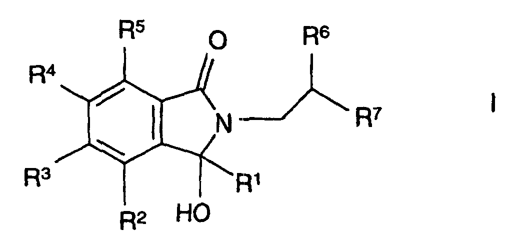 où
R¹ représente un cycloalkyle en C₃-C₇, un phényle ou le reste d'un hétérocycle aromatique à 5 ou 6 membres qui contient un ou plusieurs hétéroatomes identiques ou différents de la série N, O, S, étant entendu que le reste phényle et le reste hétérocyclique sont non substitués ou substitués par un ou plusieurs restes identiques ou différents de la série alkyle en C₁-C₄, halogène, hydroxy, alcoxy en C₁-C₄, benzyloxy, phénoxy, benzyle, phényle, trifluorométhyle, cyan, hydroxycarbonyle, (alcoxy en C₁-C₄)carbonyle, aminocarbonyle, nitro, amino, (alkyl en C₁-C₄)amino, (alkyl en C₁-C₄)amino, di-(alkyl en C₁-C₄)amino et (alkyl en C₁-C₄)carbonylamino ;
R², R³, R⁴ et R⁵ représentent indépendamment l'un de l'autre un hydrogène, un halogène, un alkyle en C₁-C₄ ou un alcoxy en C₁-C₄ ;
R⁶ représente un phényle non substitué ou substitué par un ou plusieurs restes identiques ou différents de la série alkyle en C₁-C₄, halogène, hydroxy, alcoxy en C₁-C₄, benzyloxy, phénoxy, benzyle, phényle, trifluorométhyle, cyan, hydroxycarbonyle, (alcoxy en C₁-C₄)carbonyle, aminocarbonyle, nitro, amino, (alkyl en C₁-C₄)amino, di(alkyl en C₁-C₄)amino et (alkyl en C₁-C₄)carbonylamino ;
R⁷ indépendamment de R⁶ a une des significations de R⁶ ou représente un hydrogène ;
sous toutes leurs formes stéréoisomères et leurs mélanges dans toutes les proportions, et leurs sels physiologiquement compatibles ;
étant entendu que, lorsque R¹ représente un phényle non substitué et R², R³, R⁴, R⁵ et R⁷ représentent un hydrogène, R⁶ ne peut pas simultanément représenter un phényle non substitué ou un 3,4-diméthoxyphényle.

2. Composés de formule I selon la revendication 1, où R¹ représente un phényle non substitué ou un phényle substitué par un ou deux restes identiques ou différents de la série alkyle en C₁-C₄ et halogène, sous toutes leurs formes stéréoisomères et leurs mélanges dans toutes les proportions, et leurs sels physiologiquement compatibles.

3. Composés de formule I selon les revendications 1 et/ou 2, où R², R³, R⁴ et R⁵ représentent indépendamment l'un de l'autre un hydrogène, un fluor, un chlore ou un méthyle, sous toutes leurs formes stéréoisomères et leurs mélanges dans toutes les proportions, et leurs sels physiologiquement compatibles.

4. Composés de formule I selon une ou plusieurs des revendications 1 à 3, où R⁶ représente un phényle non substitué ou substitué par un ou deux restes identiques ou différents de la série alkyle en C₁-C₄, halogène et alcoxy en C₁-C₄, sous toutes leurs formes stéréoisomères et leurs mélanges dans toutes les proportions, et leurs sels physiologiquement compatibles.

5. Composés de formule I selon une ou plusieurs des revendications 1 à 4, où R⁶ représente un phényle substitué par un ou deux restes identiques ou différents de la série halogène et alcoxy en C₁-C₄, sous toutes leurs formes stéréoisomères et leurs mélanges dans toutes les proportions, et leurs sels physiologiquement compatibles.

6. Composés de formule I selon une ou plusieurs des revendications 1 à 5, où R⁷ représente un hydrogène, sous toutes leurs formes stéréoisomères et leurs mélanges dans toutes les proportions, et leurs sels physiologiquement compatibles.

7. Composés de formule I selon une ou plusieurs des revendications 1 à 6, où
R¹ représente un phényle non substitué ou un fluorophényle ;
R², R³, R⁴ et R⁵ représentent un hydrogène ;
R⁶ représente un méthoxyphényle ou un chlorophényle ;
R⁷ représente un hydrogène ;
sous toutes leurs formes stéréoisomères et leurs mélanges dans toutes les proportions, et leurs sels physiologiquement compatibles.

8. Procédé de préparation de composés de formule I selon une ou plusieurs des revendications 1 à 7, **caractérisé en ce qu'**on met à réagir un acide benzoïque de formule II ou un dérivé activé de ce dernier avec une amine de formule III ou que l'on met à réagir un phtalimide de formule IV avec un composé organométallique de formule V, 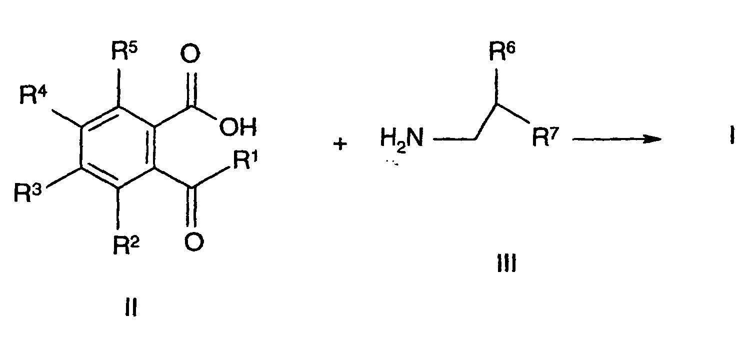 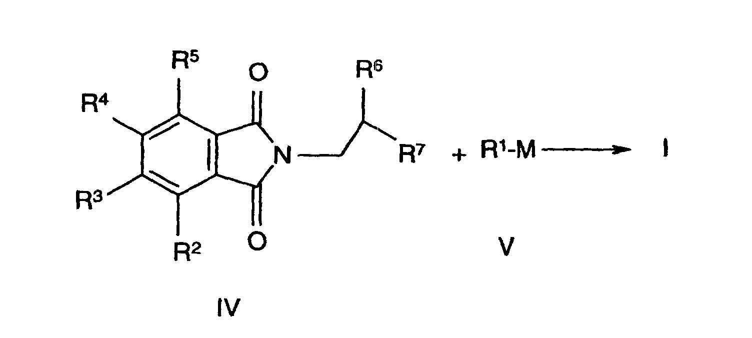 étant entendu que, dans les formules II, III et V, les restes R¹, R², R³, R⁴, R⁵, R⁶ et R⁷ ont les significations indiquées dans les revendications 1 à 7 et M représente Li ou MgCI, MgBr ou MgI.

9. Composés de formule I selon une ou plusieurs des revendications 1 à 7, et/ou leurs sels physiologiquement compatibles pour utilisation comme médicaments.

10. Préparation pharmaceutique, **caractérisée en ce qu'**elle contient un ou plusieurs composés de formule I selon une ou plusieurs des revendications 1 à 7 et/ou leurs sels physiologiquement compatibles et un support pharmaceutiquement parfait.

11. Utilisation de composés de formule I selon une ou plusieurs des revendications 1 à 7 et/ou de leurs sels physiologiquement compatibles pour la préparation d'un médicament destiné à activer la guanylate-cyclase soluble.

12. Utilisation de composés de formule I selon une ou plusieurs des revendications 1 à 7 et/ou de leurs sels physiologiquement compatibles pour la préparation d'un médicament destiné au traitement ou à la prévention des maladies cardiovasculaires, des dysfonctionnements endothéliaux, des dysfonctionnements diastoliques, de l'athérosclérose, de l'hypertension artérielle, de l'angine de poitrine, des thromboses, des resténoses, de l'infarctus myocardique, de l'ictus, de l'insuffisance cardiaque, de l'hypertension pulmonaire, des dysfonctionnements de l'érection, de l'asthme bronchique, de l'insuffisance rénale chronique, du diabète ou de la cirrhose du foie ou à l'amélioration d'une capacité d'apprentissage limitée ou du pouvoir de mémorisation.
